# EUROPEAN PATENT APPLICATION

(11) **EP 4 838 459 A1**
(43) Date of publication of application: **11.11.2026**
(21) Application number: 26160148.8
(22) Date of filing: 23.02.2026
(51) Int. Cl.: G06T 7/00, G06T 7/11, G16H 50/20

(54) **METHOD FOR CLASSIFYING EMERGENCY LEVEL OF ACUTE ABDOMINAL CONDITIONS USING ARTIFICIAL INTELLIGENCE MODELS**

(30) Priority: 09.05.2025 KR 20250060281; 11.02.2026 KR 20260027680
(71) Applicant: BeamWorks Inc., Daegu 41404 (KR)
(72) Inventor: KIM, Wonhwa, 42119 Daegu (KR); KIM, Jaeil, 41584 Daegu (KR); PARK, Sung Joon, 41416 Daegu (KR)
(74) Representative: Barbaro, Gaetano

(57) **Abstract**

According to one embodiment of the present disclosure, there is disclosed a method for triaging an emergency level of an abdominal disease by using an artificial intelligence model, performed by a computing device. The method may comprise: obtaining a medical image and patient information; predicting a severity of the abdominal disease by using the medical image; and triaging an emergency level based on the predicted severity and the patient information.

## Description

### CROSS-REFERENCE TO RELATED APPLICATIONS

This application claims priority to and the benefit of Korean Patent Application No. 10-2026-0027680 filed in the Korean Intellectual Property Office on February 11, 2026, the entire contents of which are incorporated herein by reference.

### TECHNICAL FIELD

The present invention relates to a technology for assisting emergency diagnosis and treatment decision-making, and more particularly, to a technology for detecting an abnormal finding of an acute abdominal disease by analyzing a medical image and patient clinical information by using an artificial intelligence model, and triaging surgical necessity and triaging an emergency level.

### BACKGROUND ART

In an emergency medical environment, rapidly and accurately determining whether a surgical emergency is required for a patient with acute abdomen has a significant effect on a prognosis of the patient. Causative diseases of acute abdomen are widely distributed from mild conditions such as appendicitis to ultra-emergency diseases such as bowel ischemia, perforation, intraperitoneal hemorrhage, and aortic dissection, and initial clinical manifestations are ambiguous, so that even a skilled specialist often has difficulty in differentiation.

In particular, a medical image such as abdominal CT is used as a key diagnostic means for confirming an abnormal finding in an abdominal cavity hemorrhage, bowel ischemia, perforation, free air, free fluid, organ injury, and the like.

However, conventional emergency CT reading tends to largely depend on visual reading and experience of a radiologist, and thus there is a limitation in that reading delay or variation in judgment among medical staff may occur in a night shift or an overloaded reading environment.

In particular, diseases such as bowel ischemia or perforation may progress to bowel necrosis and septic shock even if diagnosis is delayed by only several hours, and thus a mortality rate rapidly increases. In a night or weekend time zone or in an environment in which medical personnel are insufficient, it is difficult to receive immediate reading support from a radiologist, and thus a medical gap in which determination of whether to perform surgery or a decision for transfer is delayed may occur.

In addition, conventional artificial intelligence-based medical image analysis technologies often focus on individually detecting or triaging a specific lesion or finding, and thus there is a limitation in that such technologies are linked to an overall determination of a severity of an acute abdominal disease required in an actual clinical environment or classification of surgical necessity and triaging an emergency level of a patient. Furthermore, although determination of an emergency level necessarily requires consideration of not only medical image information but also patient clinical information such as age, vital signs, and a level of consciousness, a systematic technology capable of performing triaging by automatically integrating a result of medical image analysis and patient information has not been sufficiently provided.

For these reasons, in an actual emergency room environment, a CT reading result, patient clinical information, and subjective judgment of medical staff are used in a dispersed state, and as a result, a problem in which determination of a severity of an acute abdominal disease and surgical necessity is inconsistent or delayed has been continuously raised.

Korean Patent Application Publication No. 10-2025-0083115 discloses a vehicle emergency medical system and method for guiding triaging of an emergency patient, treatment, and hospital transfer.

### SUMMARY OF THE INVENTION

The present disclosure is directed to providing a method for detecting an abnormal finding related to an acute abdominal disease, quantitatively predicting a severity thereof, and automatically triaging an emergency level and triaging surgical necessity of a patient based on the predicted result, by integrally analyzing a medical image and patient information through an artificial intelligence model.

Meanwhile, a technical problem to be achieved by the present disclosure is not limited to the above-described technical problem, and various technical problems apparent to those skilled in the art from the following description may be included.

An exemplary embodiment of the present disclosure provides a method for triaging an emergency level of an abdominal disease by using an artificial intelligence model, which is performed by a computing device including at least one processor. The method may include: obtaining a medical image and patient information; predicting a severity of the abdominal disease by using the medical image; and triaging an emergency level based on the predicted severity and the patient information.

According to one embodiment, the patient information may include at least one of age, sex, body mass index, injury mechanism, blood pressure, heart rate, respiratory rate, oxygen saturation, or a Glasgow Coma Scale (GCS).

According to one embodiment, predicting a severity of an abdominal disease by using the medical image may comprise extracting abnormal finding feature information from the medical image by using a first artificial intelligence model, and predicting the severity based on the extracted abnormal finding feature information.

According to one embodiment, triaging an emergency level based on the predicted severity and the patient information may comprise triaging the emergency level based on the predicted severity and the patient information by using the second artificial intelligence model.

According to one embodiment, the first artificial intelligence model may include a deep learning model trained to extract feature information from a medical image, and the second artificial intelligence model may include a model trained to classify the emergency level by integrating the predicted severity and the patient information.

According to one embodiment, the first artificial intelligence model may include a model trained by using training medical images in which abnormal findings related to the abdominal disease are labeled, and the second artificial intelligence model may include a model trained by using training patient clinical information labeled with surgical necessity and emergency level grades and the severity extracted from the first artificial intelligence model.

According to one embodiment, the abnormal findings related to the abdominal disease may include at least one of intra-abdominal hemorrhage, bowel ischemia, perforation, organ injury, free air, free fluid, vascular abnormality, inflammatory change, or a mass.

According to one embodiment, the method may further comprise generating a suspected abnormal finding region map based on the predicted severity by using the first artificial intelligence model, and generating an emergency reading report by using the medical image, the patient information, the emergency level, and the suspected abnormal finding region map.

According to one embodiment, the first artificial intelligence model may include: an encoder configured to extract abnormal finding feature information from the medical image; a classification module configured to calculate the severity based on the extracted abnormal finding feature information; and a calculation module configured to generate the suspected abnormal finding region map based on the calculated severity.

According to one embodiment, generating the emergency reading report by using the medical image, the patient information, and the suspected abnormal finding region map may comprise displaying the suspected abnormal finding region map in an overlapped manner on the medical image in a form of a heatmap using color or contour, and additionally displaying the severity, the emergency level, and the surgical necessity.

According to one embodiment, the method may comprise detecting a modification to the patient information while the emergency reading report is displayed; retriaging the emergency level based on the severity and the modified patient information; and dynamically reflecting the reclassified emergency level on the report in response to the modification to the patient information.

According to one embodiment, predicting the severity of the abdominal disease by using the medical image may further comprise segmenting anatomical structures from the medical image, and analyzing the abnormal finding feature information with weights assigned to regions corresponding to the segmented anatomical structures by using the first artificial intelligence model.

According to one embodiment, the first artificial intelligence model may be configured to apply an attention mechanism to a segmentation result of the anatomical structures and to analyze the abnormal finding feature information by assigning weights to regions corresponding to the anatomical structures related to the abnormal findings.

According to one embodiment, the medical image may include three-dimensional volume data composed of a plurality of slices, and the first artificial intelligence model may be configured to detect the abnormal findings by learning a spatial context of the three-dimensional volume data.

According to one embodiment, triaging the emergency level based on the predicted severity and the patient information may comprise triaging into one of a surgical emergency grade or a conservative treatment possible grade.

Another exemplary embodiment of the present disclosure provides computer program stored in a computer-readable storage medium. When the computer program is executed by one or more processors, the computer program causes the one or more processors to perform operations for triaging an emergency level of an abdominal disease by using an artificial intelligence model, and the operations may comprise: obtaining a medical image and patient information; predicting a severity of the abdominal disease by using the medical image; and triaging an emergency level based on the predicted severity and the patient information.

According to one embodiment, the patient information may include at least one of age, sex, body mass index, injury mechanism, blood pressure, heart rate, respiratory rate, oxygen saturation, or a Glasgow Coma Scale (GCS).

According to one embodiment, the operation of predicting the severity of the abdominal disease by using the medical image may comprise extracting abnormal finding feature information from the medical image by using a first artificial intelligence model, and predicting the severity based on the extracted abnormal finding feature information.

According to one embodiment, the operation of triaging the emergency level based on the predicted severity and the patient information may comprise triaging the emergency level based on the predicted severity and the patient information by using the second artificial intelligence model.

According to one embodiment, the first artificial intelligence model may include a deep learning model trained to extract feature information from the medical image, and the second artificial intelligence model may include a model trained to classify the emergency level by integrating the predicted severity and the patient information.

According to one embodiment, the first artificial intelligence model may include a model trained by using training medical images in which abnormal findings related to the abdominal disease are labeled, and the second artificial intelligence model may include a model trained by using training patient clinical information labeled with surgical necessity and emergency level grades and the severity extracted from the first artificial intelligence model.

According to one embodiment, the operations may further comprise generating a suspected abnormal finding region map based on the predicted severity by using the first artificial intelligence model, and generating an emergency reading report by using the medical image, the patient information, the emergency level, and the suspected abnormal finding region map.

According to one embodiment, the first artificial intelligence model may include: an encoder configured to extract abnormal finding feature information from the medical image; a classification module configured to calculate the severity based on the extracted abnormal finding feature information; and a calculation module configured to generate the suspected abnormal finding region map based on the calculated severity.

According to one embodiment, the operation of generating the emergency reading report by using the medical image, the patient information, and the suspected abnormal finding region map may comprise displaying the suspected abnormal finding region map in an overlapped manner on the medical image in a form of a heatmap using color or contour, and additionally displaying the severity and the emergency level.

According to one embodiment, the operations may comprise detecting a modification to the patient information while the emergency reading report is displayed; retriaging the emergency level based on the severity and the modified patient information; and dynamically reflecting the reclassified emergency level information on the report in response to the modification to the patient information.

According to one embodiment of the present disclosure for implementing the above-described objective, there is disclosed a computing device. The apparatus may include: at least one processor; and a memory, wherein the at least one processor may be configured to obtain a medical image and patient information; predict a severity of an abdominal disease by using the medical image; and triage an emergency level based on the predicted severity and the patient information.

According to one embodiment, the patient information may include at least one of age, sex, body mass index, injury mechanism, blood pressure, heart rate, respiratory rate, oxygen saturation, or a Glasgow Coma Scale (GCS).

According to one embodiment, the at least one processor may be configured to extract abnormal finding feature information from the medical image by using the first artificial intelligence model and to predict the severity based on the extracted abnormal finding feature information.

According to one embodiment, the at least one processor may be configured to classify the emergency level based on the predicted severity and the patient information by using the second artificial intelligence model.

According to one embodiment, the first artificial intelligence model may include a deep learning model trained to extract feature information from the medical image, and the second artificial intelligence model may include a model trained to classify the emergency level by integrating the predicted severity and the patient information.

According to one embodiment, the first artificial intelligence model may include a model trained by using training medical images in which abnormal findings related to the abdominal disease are labeled, and the second artificial intelligence model may include a model trained by using training patient clinical information labeled with surgical necessity and emergency level grades and the severity extracted from the first artificial intelligence model.

According to one embodiment, the at least one processor may be further configured to generate a suspected abnormal finding region map based on the predicted severity by using the first artificial intelligence model, and to generate an emergency reading report by using the medical image, the patient information, the emergency level, and the suspected abnormal finding region map.

According to one embodiment, the first artificial intelligence model may include: an encoder configured to extract abnormal finding feature information from the medical image; a classification module configured to calculate the severity based on the extracted abnormal finding feature information; and a calculation module configured to generate the suspected abnormal finding region map based on the calculated severity.

According to one embodiment, the at least one processor may be configured to display the suspected abnormal finding region map in an overlapped manner on the medical image in a form of a heatmap using color or contour, and to additionally display the severity and the emergency level.

According to one embodiment, the at least one processor may be further configured to detect a modification to the patient information while the emergency reading report is displayed; reclassify the emergency level based on the severity and the modified patient information; and dynamically reflect the reclassified emergency level on the report in response to the modification to the patient information.

The present disclosure may provide an effect of improving efficiency of surgical decision-making and performing rapid and consistent triaging of an emergency patient, by enabling determination of an emergency level in which a severity prediction result for various acute abdominal diseases and patient clinical information are integrally combined in real time, through linking a first artificial intelligence model that detects an abnormal finding related to an acute abdominal disease from a medical image and predicts a severity thereof, and a second artificial intelligence model that classifies surgical necessity and triages an emergency level by using the predicted severity and patient information as inputs.

In addition, the present disclosure may achieve a high detection accuracy even for a disease in which a subtle imaging change is important, such as bowel ischemia or perforation, by segmenting a location of an anatomical structure and analyzing by assigning a weight to a corresponding region, and may provide explainable grounds for determination to medical staff by visualizing a suspected abnormal finding region.

In addition, the present disclosure may provide an effect of maintaining consistent performance even in various medical environments in which imaging equipment or protocols contrast or non-contrast are different, by using a foundation model pre-trained with large-scale medical image.

Meanwhile, effects of the present disclosure are not limited to the above-described effects, and various effects apparent to those skilled in the art from the following description may be included.

### BRIEF DESCRIPTION OF THE DRAWINGS

FIG. 1 is a block diagram of a computing device for triaging an emergency level of an abdominal disease by using an artificial intelligence model according to an embodiment of the present disclosure.
FIG. 2 is a conceptual view illustrating a neural network according to an exemplary embodiment of the present disclosure.
FIG. 3 is a flowchart illustrating a method for triaging an emergency level of an abdominal disease by using an artificial intelligence model according to an embodiment of the present disclosure.
FIG. 4 is a schematic diagram illustrating an operation of triaging an emergency level of an abdominal disease by using an artificial intelligence model according to an embodiment of the present disclosure.
FIG. 5 is a diagram schematically illustrating a result of generating an emergency reading report according to an embodiment of the present disclosure.
FIG. 6 is a normal and schematic view of an exemplary computing environment in which the exemplary embodiments of the present disclosure may be implemented.

### DETAILED DESCRIPTION

Various exemplary embodiments will now be described with reference to drawings. In the present specification, various descriptions are presented to provide appreciation of the present disclosure. However, it is apparent that the exemplary embodiments can be executed without the specific description.

"Component", "module", "system", and the like which are terms used in the specification refer to a computer-related entity, hardware, firmware, software, and a combination of the software and the hardware, or execution of the software. For example, the component may be a processing procedure executed on a processor, the processor, an object, an execution thread, a program, and/or a computer, but is not limited thereto. For example, both an application executed in a computing device and the computing device may be the components. One or more components may reside within the processor and/or a thread of execution. One component may be localized in one computer. One component may be distributed between two or more computers. Further, the components may be executed by various computer-readable media having various data structures, which are stored therein. The components may perform communication through local and/or remote processing according to a signal (for example, data transmitted from another system through a network such as the Internet through data and/or a signal from one component that interacts with other components in a local system and a distribution system) having one or more data packets, for example.

The term "or" is intended to mean not exclusive "or" but inclusive "or". That is, when not separately specified or not clear in terms of a context, a sentence "X uses A or B" is intended to mean one of the natural inclusive substitutions. That is, the sentence "X uses A or B" may be applied to any of the case where X uses A, the case where X uses B, or the case where X uses both A and B. Further, it should be understood that the term "and/or" used in this specification designates and includes all available combinations of one or more items among enumerated related items.

It should be appreciated that the term "comprise" and/or "comprising" means presence of corresponding features and/or components. However, it should be appreciated that the term "comprises" and/or "comprising" means that presence or addition of one or more other features, components, and/or a group thereof is not excluded. Further, when not separately specified or it is not clear in terms of the context that a singular form is indicated, it should be construed that the singular form generally means "one or more" in this specification and the claims.

The term "at least one of A or B" should be interpreted to mean "a case including only A", "a case including only B", and "a case in which A and B are combined".

Those skilled in the art need to recognize that various illustrative logical blocks, configurations, modules, circuits, means, logic, and algorithm steps described in connection with the exemplary embodiments disclosed herein may be additionally implemented as electronic hardware, computer software, or combinations of both sides. To clearly illustrate the interchangeability of hardware and software, various illustrative components, blocks, configurations, means, logic, modules, circuits, and steps have been described above generally in terms of their functionalities. Whether the functionalities are implemented as the hardware or software depends on a specific application and design restrictions given to an entire system. Skilled artisans may implement the described functionalities in various ways for each particular application. However, such implementation decisions should not be interpreted as causing a departure from the scope of the present disclosure.

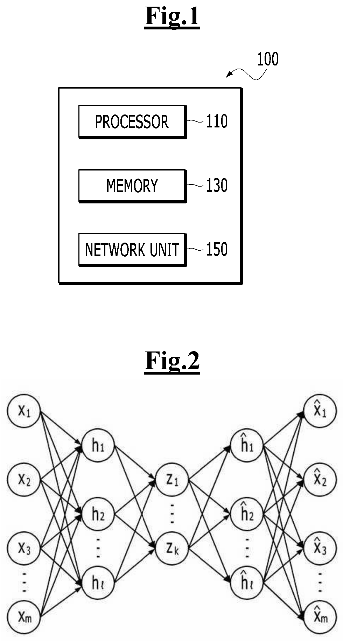

FIG. 1 is a block diagram of a computing device for triaging an emergency level of an abdominal disease by using an artificial intelligence model according to an embodiment of the present disclosure.

A configuration of the computing device 100 illustrated in FIG. 1 is only an example shown through simplification. In an exemplary embodiment of the present disclosure, the computing device 100 may include other components for performing a computing environment of the computing device 100 and only some of the disclosed components may constitute the computing device 100. The computing device 100 may include a processor 110, a memory 130, and a network unit 150.

The processor 110 may be constituted by one or more cores and may include processors for data analysis and deep learning, which include a central processing unit (CPU), a general purpose graphics processing unit (GPGPU), a tensor processing unit (TPU), and the like of the computing device. The processor 110 may read a computer program stored in the memory 130 to perform data processing for machine learning according to an exemplary embodiment of the present disclosure. According to an exemplary embodiment of the present disclosure, the processor 110 may perform a calculation for training the neural network. The processor 110 may perform calculations for training the neural network, which include processing of input data for training in deep learning (DL), extracting a feature in the input data, calculating an error, updating a weight of the neural network using backpropagation, and the like. At least one of the CPU, GPGPU, and TPU of the processor 110 may process training of a network function. For example, both the CPU and the GPGPU may process the training of the network function and data classification using the network function. Further, in an exemplary embodiment of the present disclosure, processors of a plurality of computing devices may be used together to process the training of the network function and the data classification using the network function. Further, the computer program executed in the computing device according to an exemplary embodiment of the present disclosure may be a CPU, GPGPU, or TPU executable program.

According to an exemplary embodiment of the present disclosure, the memory 130 may store any type of information generated or determined by the processor 110 and any type of information received by the network unit 150.

According to an exemplary embodiment of the present disclosure, the memory 130 may include at least one type of storage medium of a flash memory type storage medium, a hard disk type storage medium, a multimedia card micro type storage medium, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. The computing device 100 may operate in connection with a web storage performing a storing function of the memory 130 on the Internet. The description of the memory is just an example and the present disclosure is not limited thereto.

The network unit 150 according to an embodiment of the present disclosure may use various wired communication systems such as Public Switched Telephone Network PSTN, x Digital Subscriber Line xDSL, Rate Adaptive DSL RADSL, Multi Rate DSL MDSL, Very High Speed DSL VDSL, Universal Asymmetric DSL UADSL, High Bit Rate DSL HDSL, and a Local Area Network LAN.

In addition, the network unit 150 presented in the present specification may use various wireless communication systems such as Code Division Multi Access CDMA, Time Division Multi Access TDMA, Frequency Division Multi Access FDMA, Orthogonal Frequency Division Multi Access OFDMA, Single Carrier-FDMA SC-FDMA, and other systems.

In the present disclosure, the network unit 150 may be configured regardless of a communication aspect, such as wired communication and wireless communication, and may be configured by various communication networks, such as a Personal Area Network (PAN) and a Wide Area Network (WAN). Further, the network may be a publicly known World Wide Web (WWW), and may also use a wireless transmission technology used in short range communication, such as Infrared Data Association (IrDA) or Bluetooth.

The technologies described in the present specification may be used not only in the above-mentioned networks but also in other networks.

FIG. 2 is a conceptual view illustrating a neural network according to an exemplary embodiment of the present disclosure.

Throughout the present specification, a computation model, the neural network, a network function, and the neural network may be used as the same meaning. The neural network may be generally constituted by an aggregate of calculation units which are mutually connected to each other, which may be called nodes. The nodes may also be called neurons. The neural network is configured to include one or more nodes. The nodes (alternatively, neurons) constituting the neural networks may be connected to each other by one or more links.

In the neural network, one or more nodes connected through the link may relatively form the relationship between an input node and an output node. Concepts of the input node and the output node are relative and a predetermined node which has the output node relationship with respect to one node may have the input node relationship in the relationship with another node and vice versa. As described above, the relationship of the input node to the output node may be generated based on the link. One or more output nodes may be connected to one input node through the link and vice versa.

In the relationship of the input node and the output node connected through one link, a value of data of the output node may be determined based on data input in the input node. Here, a link connecting the input node and the output node to each other may have a weight. The weight may be variable and the weight is variable by a user or an algorithm in order for the neural network to perform a desired function. For example, when one or more input nodes are mutually connected to one output node by the respective links, the output node may determine an output node value based on values input in the input nodes connected with the output node and the weights set in the links corresponding to the respective input nodes.

As described above, in the neural network, one or more nodes are connected to each other through one or more links to form a relationship of the input node and output node in the neural network. A characteristic of the neural network may be determined according to the number of nodes, the number of links, correlations between the nodes and the links, and values of the weights granted to the respective links in the neural network. For example, when the same number of nodes and links exist and there are two neural networks in which the weight values of the links are different from each other, it may be recognized that two neural networks are different from each other.

The neural network may be constituted by a set of one or more nodes. A subset of the nodes constituting the neural network may constitute a layer. Some of the nodes constituting the neural network may constitute one layer based on the distances from the initial input node. For example, a set of nodes of which distance from the initial input node is n may constitute n layers. The distance from the initial input node may be defined by the minimum number of links which should be passed through for reaching the corresponding node from the initial input node. However, a definition of the layer is predetermined for description and the order of the layer in the neural network may be defined by a method different from the aforementioned method. For example, the layers of the nodes may be defined by the distance from a final output node.

The initial input node may mean one or more nodes in which data is directly input without passing through the links in the relationships with other nodes among the nodes in the neural network. Alternatively, in the neural network, in the relationship between the nodes based on the link, the initial input node may mean nodes which do not have other input nodes connected through the links. Similarly thereto, the final output node may mean one or more nodes which do not have the output node in the relationship with other nodes among the nodes in the neural network. Further, a hidden node may mean nodes constituting the neural network other than the initial input node and the final output node.

In the neural network according to an exemplary embodiment of the present disclosure, the number of nodes of the input layer may be the same as the number of nodes of the output layer, and the neural network may be a neural network of a type in which the number of nodes decreases and then, increases again from the input layer to the hidden layer. Further, in the neural network according to another exemplary embodiment of the present disclosure, the number of nodes of the input layer may be smaller than the number of nodes of the output layer, and the neural network may be a neural network of a type in which the number of nodes decreases from the input layer to the hidden layer. Further, in the neural network according to yet another exemplary embodiment of the present disclosure, the number of nodes of the input layer may be larger than the number of nodes of the output layer, and the neural network may be a neural network of a type in which the number of nodes increases from the input layer to the hidden layer. The neural network according to still yet another exemplary embodiment of the present disclosure may be a neural network of a type in which the neural networks are combined.

A deep neural network (DNN) may refer to a neural network that includes a plurality of hidden layers in addition to the input and output layers. When the deep neural network is used, the latent structures of data may be determined. That is, latent structures of photos, text, video, voice, and music (e.g., what objects are in the photo, what the content and feelings of the text are, what the content and feelings of the voice are) may be determined. The deep neural network may include a convolutional neural network (CNN), a recurrent neural network (RNN), an auto encoder, a restricted Boltzmann machine (RBM), a deep belief network (DBN), a Q network, a U network, a Siam network, a Generative Adversarial Network (GAN), Transformer, and the like. The description of the deep neural network described above is just an example and the present disclosure is not limited thereto.

The artificial intelligence-based model of the present disclosure may be represented by a network structure of any of the aforementioned structures including an input layer, a hidden layer, and an output layer.

The neural network may be trained in at least one scheme of supervised learning, unsupervised learning, semi supervised learning, transfer learning, active learning, or reinforcement learning. The learning of the neural network may be a process in which the neural network applies knowledge for performing a specific operation to the neural network.

The neural network may be trained in a direction to minimize errors of an output. The training of the neural network is a process of repeatedly inputting training data into the neural network and calculating the output of the neural network for the training data and the error of a target and back-propagating the errors of the neural network from the output layer of the neural network toward the input layer in a direction to reduce the errors to update the weight of each node of the neural network. In the case of the supervised learning, the training data labeled with a correct answer is used for each training data (i.e., the labeled training data) and in the case of the unsupervised learning, the correct answer may not be labeled in each training data. That is, for example, the training data in the case of the supervised learning related to the data classification may be data in which category is labeled in each training data. The labeled training data is input to the neural network, and the error may be calculated by comparing the output (category) of the neural network with the label of the training data. As another example, in the case of the unsupervised learning related to the data classification, the training data as the input is compared with the output of the neural network to calculate the error. The calculated error is back-propagated in a reverse direction (i.e., a direction from the output layer toward the input layer) in the neural network and connection weights of respective nodes of each layer of the neural network may be updated according to the back propagation. A variation amount of the updated connection weight of each node may be determined according to a learning rate. Calculation of the neural network for the input data and the back-propagation of the error may constitute a training cycle (epoch). The learning rate may be applied differently according to the number of repetition times of the training cycle of the neural network. For example, in an initial stage of the training of the neural network, the neural network ensures a certain level of performance quickly by using a high learning rate, thereby increasing efficiency and uses a low learning rate in a latter stage of the training, thereby increasing accuracy.

In training of the neural network, the training data may be generally a subset of actual data (i.e., data to be processed using the trained neural network), and as a result, there may be a training cycle in which errors for the training data decrease, but the errors for the actual data increase. Overfitting is a phenomenon in which the errors for the actual data increase due to excessive training of the training data. For example, a phenomenon in which the neural network that trains a cat by showing a yellow cat sees a cat other than the yellow cat and does not recognize the corresponding cat as the cat may be a kind of overfitting. The overfitting may act as a cause which increases the error of the machine learning algorithm. Various optimization methods may be used in order to prevent the overfitting. In order to prevent the overfitting, a method such as increasing the training data, regularization, dropout of omitting a part of the node of the network in the process of training, utilization of a batch normalization layer, etc., may be applied.

The present disclosure relates to artificial intelligence-based diagnosis assistance software that receives an abdominal CT medical image and patient clinical information as inputs, automatically detects a region suspected of an acute abdominal disease, and predicts a severity and an emergency level triage grade to provide the same to medical personnel.

The present disclosure may quantitatively evaluate a severity related to an acute abdominal disease and automatically classify surgical necessity and triage an emergency level of a patient by integrally analyzing a CT medical image captured in an emergency room environment and patient clinical information.

Meanwhile, the present disclosure may be implemented in a form of standalone software, may receive an abdominal CT medical image in compliance with a DICOM standard which is a medical image standard protocol, and may transmit an image in which a suspected abnormal finding region is displayed and a determination result of a severity of the abdominal disease and an emergency level to a storage system or a medical information system designated by a user.

Accordingly, the present disclosure may effectively assist rapid emergency diagnosis and treatment decision-making of medical personnel by visualizing a suspected abnormal finding region in a CT medical image captured in an emergency room as a contour or color, and providing information on a severity of the abdominal disease and an emergency level.

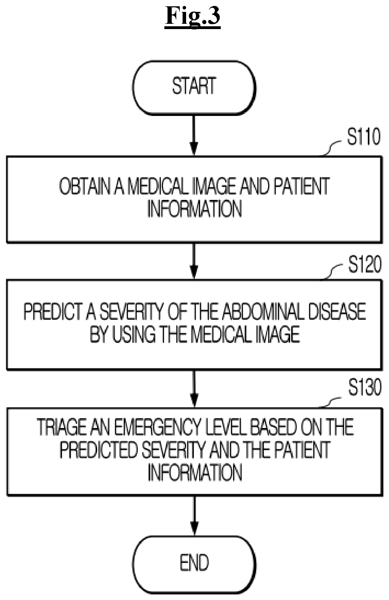

FIG. 3 is a flowchart illustrating a method for triaging an emergency level of an abdominal disease by using an artificial intelligence model according to an embodiment of the present disclosure.

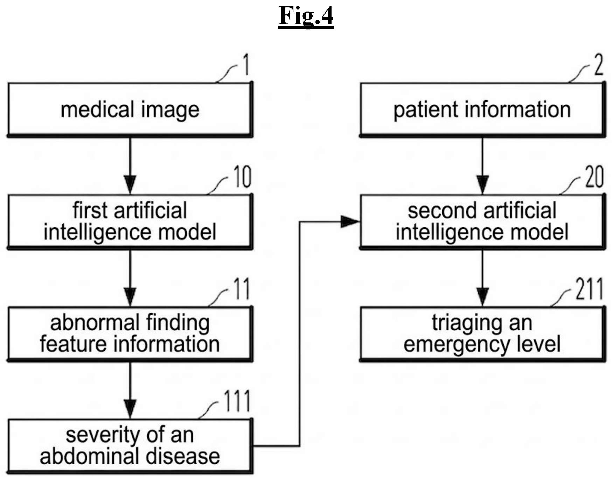

FIG. 3 is a flowchart illustrating a method for triaging an emergency level of an abdominal disease by using an artificial intelligence model according to an embodiment of the present disclosure, and FIG. 4 is a schematic diagram illustrating an operation of triaging an emergency level of an abdominal disease by using an artificial intelligence model according to an embodiment of the present disclosure.

For reference, the method for triaging an emergency level of an abdominal disease by using an artificial intelligence model may be performed by a computing device 100.

Meanwhile, in the following description, an embodiment for detecting an abnormal finding related to an acute abdominal disease, quantitatively predicting a severity thereof, and automatically triaging surgical necessity and triaging an emergency level of a patient based on the predicted result will be mainly described. However, the present disclosure is not limited thereto, and may be applied in the same or similar manner to a case of detecting an abnormal finding related to an acute disease or trauma occurring in a thorax, a head, a pelvis, a spine, or other body parts other than an abdomen, and predicting a severity and an emergency level thereof.

In this case, the medical image 1 may be an abdominal CT medical image suitable for determining an emergency level of an acute abdominal disease. The medical image 1 described below may be an abdominal CT medical image captured in an emergency environment and obtained by the computing device 100, and an operation of analyzing feature information related to an abnormal finding related to an abdominal disease and predicting a severity may be performed by applying an artificial intelligence model to the abdominal CT medical image. However, this is merely an example for convenience of description, and the present disclosure is not limited thereto and may be applied in the same or similar manner to other medical images capable of analyzing a severity of an abdominal disease.

According to an embodiment of the present disclosure, the computing device 100 may obtain a medical image 1 and patient information 2 S110. For example, the medical image 1 may be an image captured in an emergency medical environment, and may include at least one of a Computed Tomography CT image, a Magnetic Resonance Imaging MRI image, an ultrasound image, or an X-ray image. The medical image 1 may be directly received from imaging equipment in an emergency room or may be received through a network from a hospital information system PACS or a medical image storage server.

For example, the medical image 1 may include three-dimensional volume data composed of a plurality of slices, and the computing device 100 may reconstruct the plurality of slices into one continuous volume form or process the medical image in consideration of a spatial relationship between slices. For example, an abdominal CT medical image may include a plurality of cross-sectional slices continuously captured along an abdominal region of a human body, and the slices may be configured as three-dimensional volume data arranged at regular intervals.

Meanwhile, the patient information 2 may be clinical information usable for triaging an emergency level, and may include at least one of age, sex, body mass index, injury mechanism, blood pressure, heart rate, respiratory rate, oxygen saturation, or Glasgow Coma Scale GCS. The patient information 2 may be obtained through an Electronic Medical Record EMR system, an emergency medical information system, or a medical staff input interface. The computing device 100 may store the medical image and the patient information by matching the same to correspond to the same patient, and may use the same as input data for predicting a severity of an abdominal disease and triaging an emergency level performed in a subsequent step.

In another example, the patient information may include structured data and unstructured data. The structured data may include at least one of age, sex, body mass index, blood pressure, heart rate, respiratory rate, oxygen saturation, or Glasgow Coma Scale GCS. The unstructured data may include at least one of an emergency room initial record, a nursing record, or a chief complaint text.

According to an embodiment of the present disclosure, the computing device 100 may predict a presence or absence of an abnormal finding related to an acute abdominal disease and a severity of the abdominal disease by using a medical image 1 S120.

The severity of the abdominal disease may refer to an index calculated by reflecting a presence or absence of an abdominal disease or an abnormal finding observed in a corresponding patient, a progression degree thereof, or a possibility of deterioration to a clinically significant state.

The severity of the abdominal disease may be calculated by comprehensively considering imaging signs such as a type, a range, a density, a distribution, or coexistence of a plurality of abnormal findings identified in a medical image.

For example, a case in which a localized inflammatory finding is observed and a case in which extensive organ injury or a plurality of pathological signs are simultaneously observed may be evaluated as different severities.

In one embodiment, the abnormal finding related to the acute abdominal disease may include at least one of intraperitoneal hemorrhage, bowel ischemia, perforation, organ injury, free air, free fluid, vascular abnormality, inflammatory change, or mass. The computing device 100 may identify whether one or more of the abnormal findings exist from a medical image, and may predict a severity by analyzing spatial characteristics and pathological severity of each abnormal finding.

For example, the severity of the abdominal disease may be expressed in at least one form of a binary classification result for a presence or absence of an abnormal finding, a multi-level grade divided into a plurality of risk levels, or a probability value indicating a possibility of disease or complication bleeding. In addition, the severity of the abdominal disease may be an index commonly applicable to various abdominal diseases such as hemorrhage, inflammation, bowel obstruction, and perforation, may be used as input information for triaging an emergency level performed in a subsequent step, and may be provided as a reference index for assisting clinical decision-making of medical staff.

According to an embodiment of the present disclosure, the computing device 100 may preprocess a medical image 1 before predicting a presence or absence of an abnormal finding related to an acute abdominal disease and a severity of the abdominal disease. For example, the preprocessing may be a step for correcting a quality and a format of an image so that the medical image 1 is suitable to be used as an input of a first artificial intelligence model 10. For example, the computing device 100 may perform at least one of resolution adjustment, size normalization, contrast correction, noise removal, or image alignment on the medical image 1. For example, when the medical image 1 is an abdominal CT medical image, the computing device 100 may perform normalization processing on a pixel value or a voxel value of the CT medical image, or may perform windowing processing so as to be suitable for identifying a specific tissue or lesion. In addition, in the case of a CT medical image composed of a plurality of slices, the computing device 100 may correct an interval between slices or perform temporal or spatial alignment to generate a consistent input data format. The preprocessed medical image may be input to the first artificial intelligence model 10 and used so that abnormal finding feature information related to an abnormal finding related to an acute abdominal disease may be effectively extracted, and thus prediction accuracy of a severity of the abdominal disease may be improved.

According to one embodiment, the computing device 100 may extract abnormal finding feature information 11 from the medical image by using the first artificial intelligence model 10, and may predict a severity 111 of the abdominal disease based on the extracted abnormal finding feature information 11. The abnormal finding feature information 11 may include imaging signs that may be observed at a stage before a specific disease or pathological condition is clearly diagnosed in the medical image. For example, the abnormal finding feature information 11 may include at least one of a localized change in tissue density, an abnormal morphological change in a boundary of an organ or a vessel, an initial distribution pattern of free fluid, bowel wall thickening, inflammatory fat infiltration, or a fine inhomogeneity of intensity within tissue, or may include a combination thereof.

The first artificial intelligence model 10 may be configured to automatically learn and extract the abnormal finding feature information 11 from spatial patterns of the medical image. The first artificial intelligence model 10 may output a severity 111 of the abdominal disease reflecting a presence or absence of the abdominal disease or an abnormal finding in the patient, a progression degree thereof, or a possibility of deterioration to a clinically significant state, by using the abnormal finding feature information 11 as an input. The severity 111 of the abdominal disease may be expressed in a form of a continuous probability value, one of a plurality of risk levels, or a classification result for a presence or absence of the abdominal disease or an abnormal finding.

According to one embodiment, the first artificial intelligence model 10 may include a deep learning model trained to extract feature information from a medical image. For example, the first artificial intelligence model may be configured based on at least one of a Convolutional Neural Network CNN, a Transformer, a Vision Transformer ViT, a Foundation Model, or a combination thereof, but is not limited thereto. As an example, the first artificial intelligence model 10 may be configured based on a Convolutional Neural Network CNN, and in this case, may be configured to receive a preprocessed medical image as an input and hierarchically extract local image features through a plurality of convolutional layers and pooling layers. For example, the first artificial intelligence model 10 may automatically learn features such as a morphological change in an organ boundary, a localized change in tissue density, or an abnormal intensity distribution through convolution operations. In addition, the first artificial intelligence model 10 may be configured based on a Transformer or a Vision Transformer ViT structure. In this case, the first artificial intelligence model 10 may divide the medical image into a plurality of patches and learn global correlations among the patches through an attention mechanism, thereby effectively extracting inter-organ relationships or complex pathological patterns across the entire image. In addition, the first artificial intelligence model 10 may include a Foundation Model pre-trained with large-scale medical image data. The Foundation Model may be pre-trained with data specialized for an abdominal CT medical image or medical image data of various body parts and then fine-tuned according to a purpose of the present disclosure. Through this, the first artificial intelligence model 10 may stably extract abnormal finding feature information even in a limited training data environment.

According to one embodiment, the first artificial intelligence model 10 may include an encoder, a classification module, and a calculation module. This configuration reflects functional separation for calculating a severity related to an abnormal finding from a medical image and providing a result in a visually interpretable manner.

For example, the encoder may extract abnormal finding feature information 11 from the medical image. The encoder may analyze a spatial structure of the medical image and convert core features associated with an abdominal disease or an abnormal finding into a low-dimensional or high-dimensional feature vector form, and may be configured to reduce image noise and enhance representational capability of hemorrhage-related information in this process.

The classification module may calculate a severity 111 of the abdominal disease based on the extracted abnormal finding feature information 11. The classification module may be configured to receive the abnormal finding feature information 11 as an input and output a severity reflecting a presence or absence of an abdominal disease or an abnormal finding in a patient corresponding to the medical image, a progression degree thereof, or a possibility of deterioration to a clinically significant state. The severity may be expressed in one of a probability value, a multi-level grade, or a binary classification result.

The calculation module may generate a suspected abnormal finding region map based on the calculated severity 111 of the abdominal disease. The calculation module may be configured to use location information of image features having a high contribution to severity prediction and highlight a specific region on the medical image in a form of color, intensity, or contour so that medical staff can intuitively recognize a location suspected of an abnormal finding.

According to one embodiment of the present disclosure, the first artificial intelligence model 10 may include a model trained by using a training medical image labeled with an abnormal finding related to the abdominal disease. The training medical image may be an abdominal CT medical image, and each medical image may be provided in association with label information regarding a presence or absence of an abdominal disease or an abnormal finding of a patient corresponding to the medical image, a severity thereof, or a progression stage thereof.

For example, the label information may include a binary label for distinguishing a case in which an abnormal finding is present and a case in which the abnormal finding is absent in the medical image. Furthermore, the label information may include a multi-level label reflecting a severity, a range, or a clinical risk level of the abnormal finding. The first artificial intelligence model 10 may be configured to learn a correlation between imaging features related to hemorrhage included in the medical image and a severity of the abdominal disease by learning a correspondence relationship between the label information and the medical image.

In addition, the training medical image may include data of a plurality of patients collected in an actual clinical environment. By including various medical images having different imaging conditions, patient postures, image resolutions, or whether a contrast agent is used, the training medical image may allow the first artificial intelligence model 10 to be trained to stably predict a severity of the abdominal disease even under different clinical conditions.

The first artificial intelligence model 10 may repeatedly update learning parameters during a training process so as to minimize an error between abnormal finding feature information extracted from the medical image and the label information. Through this, the first artificial intelligence model 10 may be configured to precisely calculate a severity of the abdominal disease for an actual medical image input.

However, the present disclosure is not limited thereto, and the first artificial intelligence model 10 may include a model trained by using a training medical image in which various body parts are labeled. For example, the body parts may include at least one of a heart, an abdomen, a thorax, a head, a pelvis, or a limb, and accordingly, the first artificial intelligence model 10 may be trained to be applicable to medical images of various anatomical regions without being limited to a specific body part.

According to one embodiment of the present disclosure, the computing device 100 may segment a position of an anatomical structure from the medical image. The computing device 100 may identify a boundary of an anatomical structure such as an organ or a vessel in the medical image and spatially distinguish a region occupied by each anatomical structure. The anatomical structure may include at least one of an appendix, a gallbladder, a small intestine, a large intestine, an aorta, a liver, a spleen, a kidney, a pancreas, a stomach, or a vascular structure. The computing device 100 may segment a plurality of anatomical structures into independent regions. For example, in an abdominal CT medical image, the computing device 100 may generate a liver, a spleen, and a kidney in forms of mutually distinguished segmentation masks.

In addition, the computing device 100 may analyze an abnormal finding observed in a corresponding region by assigning a weight based on position information of a region corresponding to the segmented anatomical structure. The computing device 100 may combine abnormal finding feature information extracted from the medical image and a segmentation result to assign a relatively high weight to an abnormal finding observed within or adjacent to a specific anatomical structure. For example, free fluid or a change in tissue density observed in a liver or spleen region may be more strongly reflected in severity prediction.

The first artificial intelligence model 10 may be configured to apply an attention mechanism based on a segmentation result of the anatomical structures. The first artificial intelligence model 10 may use position information of the segmented anatomical structures for generating an attention map, and may assign a higher attention weight to a feature map corresponding to an anatomical region in which an abnormal finding frequently occurs. The attention mechanism may be implemented in a form of spatial attention, channel attention, or a combination thereof, and through this, the first artificial intelligence model 10 may be configured to intensively analyze an abnormal finding focusing on an anatomical region having high clinical importance instead of uniformly analyzing the entire medical image.

According to one embodiment of the present disclosure, the medical image may include three-dimensional volume data composed of a plurality of slices. The computing device 100 may spatially align the plurality of slices to configure a single continuous three-dimensional volume form. For example, an abdominal CT medical image may include a plurality of cross-sectional images captured at regular slice intervals, and the cross-sectional images may be reconstructed into three-dimensional volume data including front-rear and up-down directional information of an internal structure of a human body. The first artificial intelligence model 10 may be configured to detect an abnormal finding by learning a spatial context of the three-dimensional volume data. The first artificial intelligence model 10 may extract abnormal finding feature information by considering not only local image features limited to a single slice but also three-dimensional contextual information such as continuity between adjacent slices, relative positional relationships between organs, and spatial expansion patterns of a lesion. Through this, it is possible to more accurately detect a subtle lesion or an abnormal finding near a boundary between organs, which is difficult to identify by analysis of a single cross-sectional image.

The first artificial intelligence model 10 may include a Foundation Model pre-trained by using a large-scale medical image dataset in a self-supervised learning method. The Foundation Model may be configured to learn structural patterns of medical images even without explicit label information. For example, the Foundation Model may be trained through self-supervised learning tasks such as predicting a relationship between slices in three-dimensional volume data, restoring a partial volume region, or learning spatial consistency. In addition, the Foundation Model may include a model that inherently learns a three-dimensional anatomical structure of a human body and spatial relationships between organs. The Foundation Model may be configured to be suitable for detecting an abnormal finding by being fine-tuned for an abdominal CT medical image based on a pre-training result. Through this, the first artificial intelligence model 10 may effectively detect an abnormal finding related to an abdominal disease while maintaining stable performance even in an environment with a limited number of labeled training data.

According to another embodiment of the present disclosure, the computing device 100 may identify one or more abnormal finding from the medical image 1 by using the first artificial intelligence model. The medical image may be an abdominal CT medical image captured in an emergency environment, and the computing device 100 may identify at least one abnormal finding among intra-abdominal hemorrhage, pneumoperitoneum, bowel obstruction, bowel wall thickening, bowel perforation, acute appendicitis, acute diverticulitis, acute pancreatitis, ascites, and fat stranding by analyzing imaging features included in the medical image. The computing device 100 may identify each abnormal finding individually or determine whether a plurality of abnormal findings exist simultaneously, and may generate basic information for distinguishing a lesion due to trauma and a non-traumatic disease.

In another example, the computing device 100 may identify an abnormal finding related to an acute abdominal disease by using the medical image. The medical image may be an abdominal CT medical image captured in an emergency environment, and the computing device 100 may identify a possibility of presence of at least one of acute abdominal diseases such as appendicitis, diverticulitis, and cholecystitis by analyzing imaging features included in the medical image. In addition, the computing device 100 may identify general signs associated with the acute abdominal disease from the medical image. For example, the general signs may include at least one of inflamed fat, bowel wall thickening, ileus, free fluid, or free air.

The computing device 100 may generate basic information for distinguishing a clinically significant abnormal finding and a relatively mild non-specific acute abdominal disease by using a combination of identification results of the acute abdominal disease and the general signs. Such distinction results may be used as auxiliary information for more precisely predicting a severity of the abdominal disease. In addition, the identification results of the acute abdominal disease and the general signs may be included as part of abnormal finding feature information 11. The identification results of the acute abdominal disease and the general signs may be used as reference information in a step of calculating a severity of the abdominal disease performed by the first artificial intelligence model and a step of triaging surgical necessity and triaging an emergency level by the second artificial intelligence model. Through this, the computing device 100 may support more reliable clinical decision-making by comprehensively reflecting a plurality of pathological clues rather than being limited to a single imaging finding.

According to one embodiment of the present disclosure, the computing device 100 may generate a suspected abnormal finding region map based on a predicted severity of the abdominal disease by using the first artificial intelligence model. The first artificial intelligence model 10 may be configured to receive a preprocessed CT medical image as an input and output a severity index indicating a severity of the abdominal disease together with a suspected abnormal finding region map indicating a spatial region in the medical image where an abnormal finding is suspected. Here, the severity of the abdominal disease may be an index obtained by quantifying a possibility that an abdominal disease or a pathological abnormal finding exists or occurs in a patient corresponding to the medical image in a form of a probability value, a continuous numerical value, or one of a plurality of risk levels.

For example, the suspected abnormal finding region map may be a map spatially indicating a region determined to have a relatively high possibility that an abnormal finding exists in the medical image. The suspected abnormal finding region map may be generated by considering the severity 111 of the abdominal disease calculated by the first artificial intelligence model 10 and position information of the abnormal finding feature information 11 together.

For example, the computing device 100 may identify a pixel or voxel region having a high contribution to severity prediction by analyzing an internal feature map or an output result of the first artificial intelligence model 10. In addition, the computing device 100 may generate the suspected abnormal finding region map by mapping the same to a coordinate system of the medical image. The suspected abnormal finding region map may be configured to emphasize a region having a relatively high severity of the abdominal disease. For example, the suspected abnormal finding region map may be represented on the medical image by using at least one of visual elements of color, intensity, transparency, or contour. In the suspected abnormal finding region map, a region having a high possibility of an abnormal finding may be displayed in a relatively strong color or a clear contour. On the other hand, in the suspected abnormal finding region map, a region having a low possibility of an abnormal finding may be displayed in a weak color or a blurred contour. The computing device 100 may support medical staff to intuitively recognize a location of an abnormal finding related to the abdominal disease during a medical image reading process by displaying the suspected abnormal finding region map by overlapping the same on the original medical image. The suspected abnormal finding region map may be used as a component of an emergency reading report generated in a subsequent step.

According to one embodiment of the present disclosure, the computing device 100 may classify surgical necessity and triage an emergency level based on a severity 111 of the abdominal disease and the patient information 2 S130. The computing device 100 may determine urgency or priority of treatment required for a corresponding patient by comprehensively considering the severity of the abdominal disease calculated through medical image analysis and the patient information indicating a clinical condition of the patient. For example, the surgical necessity may refer to an index indicating whether surgery or an invasive intervention is required for an acute abdominal disease or an abnormal finding related thereto and a degree of urgency thereof. The surgical necessity may be calculated by comprehensively considering the severity of the abdominal disease predicted through medical image analysis and the clinical condition of the patient. The surgical necessity may be classified into one of a state requiring immediate surgery or intervention, a state in which surgical intervention is considered within a short period of time, or a state in which non-surgical treatment or observation may be primarily possible. For example, when an abnormal finding expected to worsen a prognosis if delayed is identified, such as perforation, extensive bowel ischemia, progressive organ injury, or a significant pathological change in an abdominal cavity, the surgical necessity may be determined as a high state. In addition, the surgical necessity may be expressed in a form of a binary decision value surgery required or not required, a grade value divided into a plurality of levels, or a continuous numerical value.

For example, the computing device 100 may classify a patient into one of a surgical emergency grade or a conservative treatment possible grade. For example, the computing device 100 may classify the patient into the surgical emergency grade when the severity of the abdominal disease is relatively high or when, even if the severity is at a medium level, vital signs or a level of consciousness of the patient are unstable. Conversely, when the severity of the abdominal disease is low and the clinical condition of the patient is stable, the patient may be classified into the conservative treatment possible grade. For example, the computing device 100 may classify the patient into a higher emergency level when the severity of the abdominal disease is relatively high or when, even if the severity is at a medium level, vital signs or a level of consciousness of the patient are unstable. Conversely, when the severity of the abdominal disease is low and the clinical condition of the patient is stable, the computing device 100 may classify the patient into a relatively lower emergency level. As described above, the emergency level may be determined by reflecting not only the severity of the abdominal disease but also an overall clinical condition of the patient.

For example, the surgical emergency grade may include a case in which immediate surgery or intervention is determined to be required. The conservative treatment possible grade may include a case in which treatment is determined to be possible by medication such as antibiotics, observation, or other non-surgical treatment methods.

In addition, the emergency level may be subdivided into a plurality of grades. For example, the emergency level may include a first grade corresponding to a state requiring immediate surgery, a second grade corresponding to a state requiring urgent intervention or active treatment within a short period, and a third grade corresponding to a state in which conservative treatment or observation is possible. However, the number of grades of the emergency level and classification criteria of each grade may be variously changed according to a medical environment, an application purpose, or a clinical protocol, and the present disclosure is not limited thereto.

The computing device 100 may display the classified surgical necessity and the emergency level 211 on a screen in association with the medical image, the severity of the abdominal disease, and the suspected abnormal finding region map, or may provide the same to medical staff by including the same as a component of an emergency reading report, thereby supporting determination of patient priority and establishment of a treatment plan in an emergency room environment. Such classification results of the emergency level may be used as reference information for supporting determination of patient priority in an emergency room, determination of whether to perform additional imaging, a request for consultation with a specialist, or establishment of a treatment plan. The computing device 100 may display the classified surgical necessity and the emergency level on a screen together with the medical image, the severity of the abdominal disease, and the suspected abnormal finding region map as described above. In addition, the computing device 100 may include the surgical necessity and the classified emergency level as components of an emergency reading report and provide the same to medical staff, thereby assisting rapid diagnosis and treatment decision-making in an emergency environment.

According to one embodiment of the present disclosure, the computing device 100 may classify a surgical necessity and an emergency level 211 based on a severity 111 of an abdominal disease predicted by using a second artificial intelligence model 20 and patient information 2. The second artificial intelligence model 20 may be configured to receive, as inputs, preprocessed patient clinical information 2 and severity feature information of the abdominal disease calculated by the first artificial intelligence model 10, and to output a classification result indicating an emergency level and a surgical necessity of a patient. The second artificial intelligence model 20 may be configured to analyze a combined relationship between the severity 111 of the abdominal disease and the patient information 2 such that, even when the severity is identical or similar, different emergency levels 211 are classified according to vital signs or a level of consciousness of the patient. For example, even when the severity of the abdominal disease is predicted to be at a medium level, when hypotension of the patient or a decrease in a Glasgow Coma Scale (GCS) is observed, the patient may be classified into an emergency state with a high surgical necessity.

According to one embodiment, the second artificial intelligence model 20 may include a model trained based on at least one of a multi-layer perceptron (MLP), a transformer, a graph neural network (GNN), a large language model (LLM), or a combination thereof. The second artificial intelligence model 20 may be configured to nonlinearly combine input data having different characteristics, such as the severity 111 of the abdominal disease and the patient information 2, and to convert the same into a representation suitable for classification of the emergency level 211. To this end, the second artificial intelligence model 20 may include a plurality of input nodes, at least one hidden layer, and an output layer, and the preprocessed patient clinical information and the severity feature of the abdominal disease calculated by the first artificial intelligence model 10 may be respectively input to the input nodes. In the hidden layer, a correlation relationship and a nonlinear combination relationship between the severity 111 of the abdominal disease and the patient information 2 may be learned.

The second artificial intelligence model 20 may be configured to output, at the output layer, at least one of a classification result of the emergency level or a classification result of the surgical necessity of the patient, and the classification result may be expressed in a form of one of a plurality of grades or a continuous numerical value. However, a specific neural network structure of the second artificial intelligence model 20, the number of hidden layers, the number of nodes, or training parameters may be variously modified within a range capable of achieving the objective of the present disclosure, and the present disclosure is not limited thereto.

According to one embodiment, the second artificial intelligence model 20 may include a model trained by using training patient clinical information labeled with an emergency level grade and a surgical necessity, and severity information of the abdominal disease extracted from the first artificial intelligence model 10. For example, training may be performed by a supervised learning scheme, and for this purpose, training data may be configured to include medical images, patient clinical information, and severity labels and emergency level and surgical necessity labels respectively corresponding thereto.

For example, each sample of the training data may be configured such that a medical image collected for a specific patient and clinical information of the corresponding patient correspond to each other, and the medical image may be associated with a severity label for the abdominal disease. The patient clinical information and the severity information may be associated with ground-truth labels for the emergency level and the surgical necessity. The second artificial intelligence model 20 may be configured to learn a relationship in which an appropriate emergency level and surgical necessity are assigned according to a combination of the severity of the abdominal disease and the patient clinical information by using the training data configured as described above.

During a training process, the second artificial intelligence model 20 may iteratively update training parameters so as to minimize an error between a classification result of the emergency level calculated as an output of the model and the emergency level grade label, and thus may be implemented as a trained model capable of automatically triaging the emergency level and the surgical necessity of a corresponding patient when actual patient data are input.

According to another embodiment of the present disclosure, the computing device 100 may classify an emergency level including an acuity of a patient and a surgical necessity based on a severity of an abdominal disease and patient information. The computing device 100 may calculate the emergency level indicating an urgency of treatment by comprehensively considering the severity of the abdominal disease predicted from a medical image and clinical information of the patient. The acuity may be used as an index for determining whether an immediate treatment is required for a condition of the patient. The acuity may be classified into one of Low, Not High, or High. For example, when the severity of the abdominal disease is low and vital signs of the patient are stable, the patient may be classified as Low acuity, and when the severity of the abdominal disease is high or when organ injury, organ dysfunction, or a significant abnormal finding is identified, the patient may be classified as High acuity.

In addition, the computing device 100 may calculate the acuity by considering a prediction result regarding whether an injury to abdominal organs such as a liver, a spleen, a kidney, or a bowel is present and a degree of the injury, or whether a significant abnormal finding observed in an abdominal cavity is present. In this case, even for an identical type of injury, different acuity levels may be classified depending on an extent of the injury, a type of an affected organ, or a severity. A classification result of the acuity may be used as a detailed criterion for triaging an emergency level, and may be included as information provided to medical staff in a subsequent emergency reading report generation step. Accordingly, medical staff may intuitively identify not only a type of an injury or a disease of a patient but also clinical severity of the corresponding condition and a necessity for an immediate response.

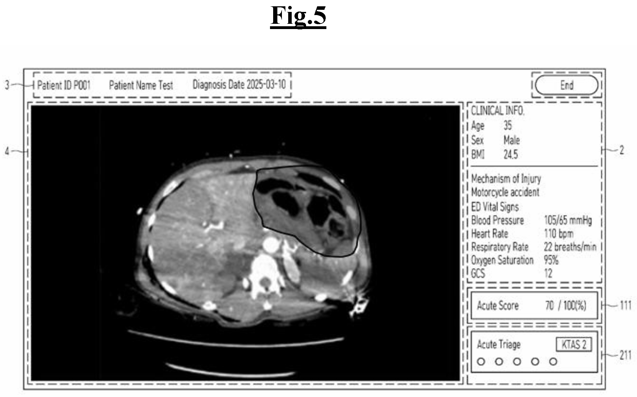

FIG. 5 is a diagram schematically illustrating a result of generating an emergency reading report according to one embodiment of the present disclosure.

According to one embodiment of the present disclosure, the computing device 100 may generate an emergency reading report by using a medical image, patient information, a severity of an abdominal disease, a surgical necessity, an emergency level, and a suspected abnormal finding region map. For example, the emergency reading report may be configured as a set of information for comprehensively providing a medical image analysis result and a clinical condition of a patient, and may include, together, a location of an abnormal finding identified in the medical image, the severity of the abdominal disease, the surgical necessity, and the emergency level classified based thereon. The computing device 100 may display the suspected abnormal finding region map in an overlapped manner on the medical image and may arrange, together, the severity, the surgical necessity, and the emergency level associated with the medical image so that medical staff may intuitively recognize a reading result.

According to one embodiment, the emergency reading report may further display, together, patient information 2 including at least one of age, sex, body mass index, injury mechanism, blood pressure, heart rate, respiratory rate, oxygen saturation, or a Glasgow Coma Scale (GCS). Accordingly, medical staff may interpret the medical image analysis result in association with the clinical condition of the patient.

The computing device 100 may display the suspected abnormal finding region map in an overlapped manner on the medical image in a form of a heatmap using at least one of color, intensity, or contour, and may be configured such that a region contributing relatively highly to the severity of the abdominal disease is visually emphasized. In addition, the severity of the abdominal disease may be displayed in a form of a numerical value, a probability value, or a risk grade, and the surgical necessity and the emergency level may be included in the report in a form of one of a plurality of grades or a continuous value.

In addition, the emergency reading report may be generated in at least one of a report for display on a screen, a report in an electronic document form, or a data form transmittable to a medical information system. The emergency reading report may be used as reference material for supporting reading in an emergency room, determining patient priority, or establishing an additional examination or treatment plan. The computing device 100 may store the emergency reading report in a storage device or transmit the same to a hospital information system or a medical image storage or transmission system, thereby supporting management of diagnostic records and collaboration in an emergency medical environment.

According to one embodiment, referring to FIG. 5, the computing device 100 may display, in a first area of an emergency reading report screen, a suspected abnormal finding region map 4 in an overlapped manner on a medical image 1 in a form of a heatmap using color or contour. For example, the medical image 1, such as an abdominal CT image, may be displayed in a central area of the screen. Accordingly, medical staff may intuitively recognize a location at which an abnormal finding is suspected during a reading process of the medical image.

In addition, the computing device 100 may display, in a second area of the emergency reading report screen, patient management information 3 including identification information of a patient and a diagnosis date. For example, the patient management information may include a patient identifier (ID), a patient name, and a diagnosis date.

In addition, the computing device 100 may display, in a third area of the emergency reading report screen, patient information 2. In an area in which the patient information 2 is displayed, emergency room vital sign information such as age, sex, body mass index (BMI), injury mechanism, blood pressure, heart rate, respiratory rate, oxygen saturation, and a Glasgow Coma Scale (GCS) of the patient may be included and displayed.

In addition, the computing device 100 may display, in a fourth area of the emergency reading report screen, a severity 111 of an abdominal disease predicted by the first artificial intelligence model. The severity 111 of the abdominal disease may be expressed in a form of a numerical value, a probability value, or a risk index. The computing device 100 may display, in the fourth area, a score (Acute Score) for the severity of the abdominal disease.

In addition, the computing device 100 may display, in a fifth area of the emergency reading report screen, a surgical necessity and an emergency level 211 classified by the second artificial intelligence model. The emergency level 211 may be expressed as one of a plurality of emergency level grades or may be provided in a visual representation including a color or an icon, such that medical staff may identify, at a glance, an urgency of treatment and whether surgery is required for a corresponding patient.

As described above, the computing device 100 may integrally display, within a single screen, the medical image 1, the suspected abnormal finding region map 4, the patient management information 3, the patient information 2, the severity 111 of the abdominal disease, and the emergency level 211, thereby improving reading efficiency of medical staff in an emergency medical environment and supporting rapid and accurate decision-making.

According to one embodiment of the present disclosure, the computing device 100 may detect a modification to the patient information while the emergency reading report is displayed. The modification to the patient information may include a change in clinical information input by medical staff, for example, a change in values of blood pressure, heart rate, respiratory rate, oxygen saturation, or a Glasgow Coma Scale (GCS). The computing device 100 may detect the modification of the patient information through an input interface on the emergency reading report screen or through a change event received from an external medical information system.

In addition, the computing device 100 may reclassify the surgical necessity and the emergency level based on the previously predicted severity 111 of the abdominal disease and the modified patient information. The computing device 100 may recalculate the surgical necessity and the emergency level by reflecting the modified patient clinical information as new input data using the second artificial intelligence model 20 while maintaining the severity 111 of the abdominal disease predicted by the first artificial intelligence model 10. In this case, when a change in a clinical condition such as deterioration of vital signs or a change in a level of consciousness of the patient is detected, the computing device 100 may reclassify a different surgical necessity or emergency level even when the severity remains the same.

In addition, the computing device 100 may dynamically reflect the reclassified surgical necessity and emergency level information on the report in response to the modification of the patient information. The computing device 100 may update and display the reclassified emergency level on the emergency reading report screen in real time. For example, the computing device 100 may change an emergency level grade, a color indication, or an icon indication so that medical staff may immediately recognize a changed patient condition. Such dynamic reflection may be performed while maintaining the medical image and the suspected abnormal finding region map, such that medical staff may continuously identify a change in the emergency level according to a change in patient clinical information within an existing reading context.

Meanwhile, the computing device 100 may support reclassification of the surgical necessity and the emergency level according to a change in patient information and dynamic display of the result, thereby effectively assisting decision-making of medical staff so as to rapidly respond to a change in a patient condition in an emergency medical environment.

The steps described above may, according to embodiments of the present disclosure, be further divided into additional steps or combined into fewer steps. In addition, some steps may be omitted as necessary, and an order of the steps may be changed.

Disclosed is a computer readable medium storing the data structure according to an exemplary embodiment of the present disclosure.

The data structure may refer to the organization, management, and storage of data that enables efficient access to and modification of data. The data structure may refer to the organization of data for solving a specific problem (e.g., data search, data storage, data modification in the shortest time). The data structures may be defined as physical or logical relationships between data elements, designed to support specific data processing functions. The logical relationship between data elements may include a connection between data elements that the user defines. The physical relationship between data elements may include an actual relationship between data elements physically stored on a computer-readable storage medium (e.g., persistent storage device). The data structure may specifically include a set of data, a relationship between the data, a function which may be applied to the data, or instructions. Through an effectively designed data structure, a computing device can perform operations while using the resources of the computing device to a minimum. Specifically, the computing device can increase the efficiency of operation, read, insert, delete, compare, exchange, and search through the effectively designed data structure.

The data structure may be divided into a linear data structure and a non-linear data structure according to the type of data structure. The linear data structure may be a structure in which only one data is connected after one data. The linear data structure may include a list, a stack, a queue, and a deque. The list may mean a series of data sets in which an order exists internally. The list may include a linked list. The linked list may be a data structure in which data is connected in a scheme in which each data is linked in a row with a pointer. In the linked list, the pointer may include link information with next or previous data. The linked list may be represented as a single linked list, a double linked list, or a circular linked list depending on the type. The stack may be a data listing structure with limited access to data. The stack may be a linear data structure that may process (e.g., insert or delete) data at only one end of the data structure. The data stored in the stack may be a data structure (LIFO-Last in First Out) in which the data is input last and output first. The queue is a data listing structure that may access data limitedly and unlike a stack, the queue may be a data structure (FIFO-First in First Out) in which late stored data is output late. The deque may be a data structure capable of processing data at both ends of the data structure.

The non-linear data structure may be a structure in which a plurality of data are connected after one data. The non-linear data structure may include a graph data structure. The graph data structure may be defined as a vertex and an edge, and the edge may include a line connecting two different vertices. The graph data structure may include a tree data structure. The tree data structure may be a data structure in which there is one path connecting two different vertices among a plurality of vertices included in the tree. That is, the tree data structure may be a data structure that does not form a loop in the graph data structure.

The data structure may include the neural network. In addition, the data structures, including the neural network, may be stored in a computer readable medium. The data structure including the neural network may also include data preprocessed for processing by the neural network, data input to the neural network, weights of the neural network, hyper parameters of the neural network, data obtained from the neural network, an active function associated with each node or layer of the neural network, and a loss function for training the neural network. The data structure including the neural network may include predetermined components of the components disclosed above. In other words, the data structure including the neural network may include all of data preprocessed for processing by the neural network, data input to the neural network, weights of the neural network, hyper parameters of the neural network, data obtained from the neural network, an active function associated with each node or layer of the neural network, and a loss function for training the neural network or a combination thereof. In addition to the above-described configurations, the data structure including the neural network may include predetermined other information that determines the characteristics of the neural network. In addition, the data structure may include all types of data used or generated in the calculation process of the neural network, and is not limited to the above. The computer readable medium may include a computer readable recording medium and/or a computer readable transmission medium. The neural network may be generally constituted by an aggregate of calculation units which are mutually connected to each other, which may be called nodes. The nodes may also be called neurons. The neural network is configured to include one or more nodes.

The data structure may include data input into the neural network. The data structure including the data input into the neural network may be stored in the computer readable medium. The data input to the neural network may include training data input in a neural network training process and/or input data input to a neural network in which training is completed. The data input to the neural network may include preprocessed data and/or data to be preprocessed. The preprocessing may include a data processing process for inputting data into the neural network. Therefore, the data structure may include data to be preprocessed and data generated by preprocessing. The data structure is just an example and the present disclosure is not limited thereto.

The data structure may include the weight of the neural network (in the present disclosure, the weight and the parameter may be used as the same meaning). In addition, the data structures, including the weight of the neural network, may be stored in the computer readable medium. The neural network may include a plurality of weights. The weight may be variable and the weight is variable by a user or an algorithm in order for the neural network to perform a desired function. For example, when one or more input nodes are mutually connected to one output node by the respective links, the output node may determine a data value output from an output node based on values input in the input nodes connected with the output node and the weights set in the links corresponding to the respective input nodes. The data structure is just an example and the present disclosure is not limited thereto.

As a non-limiting example, the weight may include a weight which varies in the neural network training process and/or a weight in which neural network training is completed. The weight which varies in the neural network training process may include a weight at a time when a training cycle starts and/or a weight that varies during the training cycle. The weight in which the neural network training is completed may include a weight in which the training cycle is completed. Accordingly, the data structure including the weight of the neural network may include a data structure including the weight which varies in the neural network training process and/or the weight in which neural network training is completed. Accordingly, the above-described weight and/or a combination of each weight are included in a data structure including a weight of a neural network. The data structure is just an example and the present disclosure is not limited thereto.

The data structure including the weight of the neural network may be stored in the computer-readable storage medium (e.g., memory, hard disk) after a serialization process. Serialization may be a process of storing data structures on the same or different computing devices and later reconfiguring the data structure and converting the data structure to a form that may be used. The computing device may serialize the data structure to send and receive data over the network. The data structure including the weight of the serialized neural network may be reconfigured in the same computing device or another computing device through deserialization. The data structure including the weight of the neural network is not limited to the serialization. Furthermore, the data structure including the weight of the neural network may include a data structure (for example, B-Tree, Trie, m-way search tree, AVL tree, and Red-Black Tree in a nonlinear data structure) to increase the efficiency of operation while using resources of the computing device to a minimum. The above-described matter is just an example and the present disclosure is not limited thereto.

The data structure may include hyper-parameters of the neural network. In addition, the data structures, including the hyper-parameters of the neural network, may be stored in the computer readable medium. The hyper-parameter may be a variable which may be varied by the user. The hyper-parameter may include, for example, a learning rate, a cost function, the number of training cycle iterations, weight initialization (for example, setting a range of weight values to be subjected to weight initialization), and Hidden Unit number (e.g., the number of hidden layers and the number of nodes in the hidden layer). The data structure is just an example and the present disclosure is not limited thereto.

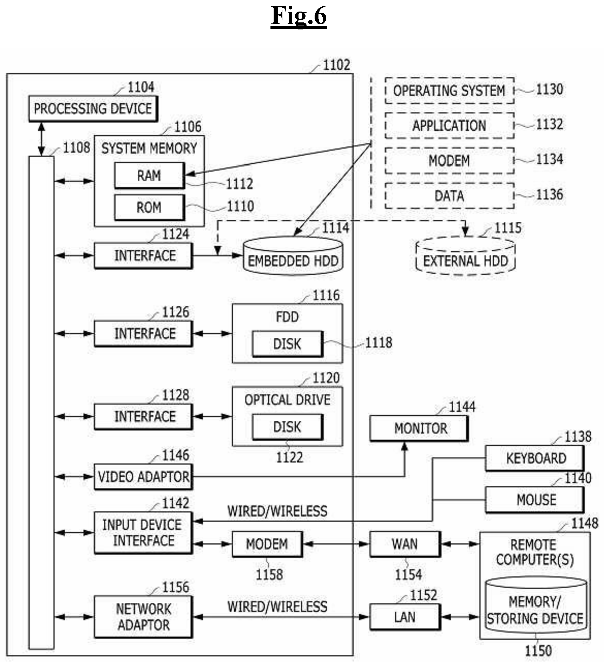

FIG. 6 is a normal and schematic view of an exemplary computing environment in which the exemplary embodiments of the present disclosure may be implemented.

It is described above that the present disclosure may be generally implemented by the computing device, but those skilled in the art will well know that the present disclosure may be implemented in association with a computer executable command which may be executed on one or more computers and/or in combination with other program modules and/or a combination of hardware and software.

In general, the program module includes a routine, a program, a component, a data structure, and the like that execute a specific task or implement a specific abstract data type. Further, it will be well appreciated by those skilled in the art that the method of the present disclosure can be implemented by other computer system configurations including a personal computer, a handheld computing device, microprocessor-based or programmable home appliances, and others (the respective devices may operate in connection with one or more associated devices) as well as a single-processor or multi-processor computer system, a mini computer, and a main frame computer.

The exemplary embodiments described in the present disclosure may also be implemented in a distributed computing environment in which predetermined tasks are performed by remote processing devices connected through a communication network. In the distributed computing environment, the program module may be positioned in both local and remote memory storage devices.

The computer generally includes various computer readable media. Media accessible by the computer may be computer readable media regardless of types thereof and the computer readable media include volatile and non-volatile media, transitory and non-transitory media, and mobile and non-mobile media. As a non-limiting example, the computer readable media may include both computer readable storage media and computer readable transmission media. The computer readable storage media include volatile and non-volatile media, transitory and non-transitory media, and mobile and non-mobile media implemented by a predetermined method or technology for storing information such as a computer readable instruction, a data structure, a program module, or other data. The computer readable storage media include a RAM, a ROM, an EEPROM, a flash memory or other memory technologies, a CD-ROM, a digital video disk (DVD) or other optical disk storage devices, a magnetic cassette, a magnetic tape, a magnetic disk storage device or other magnetic storage devices or predetermined other media which may be accessed by the computer or may be used to store desired information, but are not limited thereto.

The computer readable transmission media generally implement the computer readable command, the data structure, the program module, or other data in a carrier wave or a modulated data signal such as other transport mechanism and include all information transfer media. The term "modulated data signal" means a signal acquired by setting or changing at least one of characteristics of the signal so as to encode information in the signal. As a non-limiting example, the computer readable transmission media include wired media such as a wired network or a direct-wired connection and wireless media such as acoustic, RF, infrared and other wireless media. A combination of anymedia among the aforementioned media is also included in a range of the computer readable transmission media.

An exemplary environment 1100 that implements various aspects of the present disclosure including a computer 1102 is shown and the computer 1102 includes a processing device 1104, a system memory 1106, and a system bus 1108. The system bus 1108 connects system components including the system memory 1106 (not limited thereto) to the processing device 1104. The processing device 1104 may be a predetermined processor among various commercial processors. A dual processor and other multi-processor architectures may also be used as the processing device 1104.

The system bus 1108 may be any one of several types of bus structures which may be additionally interconnected to a local bus using any one of a memory bus, a peripheral device bus, and various commercial bus architectures. The system memory 1106 includes a read only memory (ROM) 1110 and a random access memory (RAM) 1112. A basic input/output system (BIOS) is stored in the non-volatile memories 1110 including the ROM, the EPROM, the EEPROM, and the like and the BIOS includes a basic routine that assists in transmitting information among components in the computer 1102 at a time such as in-starting. The RAM 1112 may also include a high-speed RAM including a static RAM for caching data, and the like.

The computer 1102 also includes an interior hard disk drive (HDD) 1114 (for example, EIDE and SATA), in which the interior hard disk drive 1114 may also be configured for an exterior purpose in an appropriate chassis (not illustrated), a magnetic floppy disk drive (FDD) 1116 (for example, for reading from or writing in a mobile diskette 1118), and an optical disk drive 1120 (for example, for reading a CD-ROM disk 1122 or reading from or writing in other high-capacity optical media such as the DVD, and the like). The hard disk drive 1114, the magnetic disk drive 1116, and the optical disk drive 1120 may be connected to the system bus 1108 by a hard disk drive interface 1124, a magnetic disk drive interface 1126, and an optical drive interface 1128, respectively. An interface 1124 for implementing an exterior drive includes at least one of a universal serial bus (USB) and an IEEE 1394 interface technology or both of them.

The drives and the computer readable media associated therewith provide non-volatile storage of the data, the data structure, the computer executable instruction, and others. In the case of the computer 1102, the drives and the media correspond to storing of predetermined data in an appropriate digital format. In the description of the computer readable media, the mobile optical media such as the HDD, the mobile magnetic disk, and the CD or the DVD are mentioned, but it will be well appreciated by those skilled in the art that other types of media readable by the computer such as a zip drive, a magnetic cassette, a flash memory card, a cartridge, and others may also be used in an exemplary operating environment and further, the predetermined media may include computer executable commands for executing the methods of the present disclosure.

Multiple program modules including an operating system 1130, one or more application programs 1132, other program module 1134, and program data 1136 may be stored in the drive and the RAM 1112. All or some of the operating system, the application, the module, and/or the data may also be cached in the RAM 1112. It will be well appreciated that the present disclosure may be implemented in operating systems which are commercially usable or a combination of the operating systems.

A user may input instructions and information in the computer 1102 through one or more wired/wireless input devices, for example, pointing devices such as a keyboard 1138 and a mouse 1140. Other input devices (not illustrated) may include a microphone, an IR remote controller, a joystick, a game pad, a stylus pen, a touch screen, and others. These and other input devices are often connected to the processing device 1104 through an input device interface 1142 connected to the system bus 1108, but may be connected by other interfaces including a parallel port, an IEEE 1394 serial port, a game port, a USB port, an IR interface, and others.

A monitor 1144 or other types of display devices are also connected to the system bus 1108 through interfaces such as a video adapter 1146, and the like. In addition to the monitor 1144, the computer generally includes other peripheral output devices (not illustrated) such as a speaker, a printer, others.

The computer 1102 may operate in a networked environment by using a logical connection to one or more remote computers including remote computer(s) 1148 through wired and/or wireless communication. The remote computer(s) 1148 may be a workstation, a computing device computer, a router, a personal computer, a portable computer, a micro-processor based entertainment apparatus, a peer device, or other general network nodes and generally includes multiple components or all of the components described with respect to the computer 1102, but only a memory storage device 1150 is illustrated for brief description. The illustrated logical connection includes a wired/wireless connection to a local area network (LAN) 1152 and/or a larger network, for example, a wide area network (WAN) 1154. The LAN and WAN networking environments are general environments in offices and companies and facilitate an enterprise-wide computer network such as Intranet, and all of them may be connected to a worldwide computer network, for example, the Internet.

When the computer 1102 is used in the LAN networking environment, the computer 1102 is connected to a local network 1152 through a wired and/or wireless communication network interface or an adapter 1156. The adapter 1156 may facilitate the wired or wireless communication to the LAN 1152 and the LAN 1152 also includes a wireless access point installed therein in order to communicate with the wireless adapter 1156. When the computer 1102 is used in the WAN networking environment, the computer 1102 may include a modem 1158 or has other means that configure communication through the WAN 1154 such as connection to a communication computing device on the WAN 1154 or connection through the Internet. The modem 1158 which may be an internal or external and wired or wireless device is connected to the system bus 1108 through the serial port interface 1142. In the networked environment, the program modules described with respect to the computer 1102 or some thereof may be stored in the remote memory/storage device 1150. It will be well known that an illustrated network connection is exemplary and other means configuring a communication link among computers may be used.

The computer 1102 performs an operation of communicating with predetermined wireless devices or entities which are disposed and operated by the wireless communication, for example, the printer, a scanner, a desktop and/or a portable computer, a portable data assistant (PDA), a communication satellite, predetermined equipment or place associated with a wireless detectable tag, and a telephone. This at least includes wireless fidelity (Wi-Fi) and Bluetooth wireless technology. Accordingly, communication may be a predefined structure like the network in the related art or just ad hoc communication between at least two devices.

The wireless fidelity (Wi-Fi) enables connection to the Internet, and the like without a wired cable. The Wi-Fi is a wireless technology such as the device, for example, a cellular phone which enables the computer to transmit and receive data indoors or outdoors, that is, anywhere in a communication range of a base station. The Wi-Fi network uses a wireless technology called IEEE 802.11(a, b, g, and others) in order to provide safe, reliable, and high-speed wireless connection. The Wi-Fi may be used to connect the computers to each other or the Internet and the wired network (using IEEE 802.3 or Ethernet). The Wi-Fi network may operate, for example,at a data rate of 11 Mbps (802.11a) or 54 Mbps (802.11b) in unlicensed 2.4 and 5GHz wireless bands or operate in a product including both bands (dual bands).

It will be appreciated by those skilled in the art that information and signals may be expressed by using various different predetermined technologies and techniques. For example, data, instructions, commands, information, signals, bits, symbols, and chips which may be referred in the above description may be expressed by voltages, currents, electromagnetic waves, magnetic fields or particles, optical fields or particles, or predetermined combinations thereof.

It may be appreciated by those skilled in the art that various exemplary logical blocks, modules, processors, means, circuits, and algorithm steps described in association with the exemplary embodiments disclosed herein may be implemented by electronic hardware, various types of programs or design codes (for easy description, herein, designated as software), or a combination of all of them. In order to clearly describe the intercompatibility of the hardware and the software, various exemplary components, blocks, modules, circuits, and steps have been generally described above in association with functions thereof. Whether the functions are implemented as the hardware or software depends on design restrictions given to a specific application and an entire system. Those skilled in the art of the present disclosure may implement functions described by various methods with respect to each specific application, but it should not be interpreted that the implementation determination departs from the scope of the present disclosure.

Various exemplary embodiments presented herein may be implemented as manufactured articles using a method, a device, or a standard programming and/or engineering technique. The term manufactured article includes a computer program, a carrier, or a medium which is accessible by a predetermined computer-readable storage device. For example, a computer-readable storage medium includes a magnetic storage device (for example, a hard disk, a floppy disk, a magnetic strip, or the like), an optical disk (for example, a CD, a DVD, or the like), a smart card, and a flash memory device (for example, an EEPROM, a card, a stick, a key drive, or the like), but is not limited thereto. Further, various storage media presented herein include one or more devices and/or other machine-readable media for storing information.

It will be appreciated that a specific order or a hierarchical structure of steps in the presented processes is one example of exemplary accesses. It will be appreciated that the specific order or the hierarchical structure of the steps in the processes within the scope of the present disclosure may be rearranged based on design priorities. Appended method claims provide elements of various steps in a sample order, but the method claims are not limited to the presented specific order or hierarchical structure.

The description of the presented exemplary embodiments is provided so that those skilled in the art of the present disclosure use or implement the present disclosure. Various modifications of the exemplary embodiments will be apparent to those skilled in the art and general principles defined herein can be applied to other exemplary embodiments without departing from the scope of the present disclosure. Therefore, the present disclosure is not limited to the exemplary embodiments presented herein, but should be interpreted within the widest range which is coherent with the principles and new features presented herein.

## Claims

1. A method for triaging an emergency level of an abdominal disease by using an artificial intelligence model, performed by a computing device,
the method comprising:
obtaining a medical image and patient information;
predicting a severity of the abdominal disease by using the medical image; and
triaging an emergency level based on the predicted severity and the patient information.

2. The method of claim 1, wherein predicting a severity of the abdominal disease by using the medical image comprises:
extracting abnormal finding feature information from the medical image by using a first artificial intelligence model, and predicting the severity based on the extracted abnormal finding feature information.

3. The method of claim 2, wherein triaging the emergency level based on the predicted severity and the patient information comprises:
triaging the emergency level based on the predicted severity and the patient information by using a second artificial intelligence model.

4. The method of claim 3, wherein the first artificial intelligence model comprises a deep learning model trained to extract feature information from medical image, and
wherein the second artificial intelligence model comprises a model trained to triage the emergency level by integrating the predicted severity and the patient information.

5. The method of claim 3, wherein the first artificial intelligence model comprises a model trained by using a training medical image labeled with an abnormal finding related to the abdominal disease, and
wherein the second artificial intelligence model comprises a model trained by using training patient clinical information labeled with surgical necessity and an emergency level grade, and the severity extracted from the first artificial intelligence model.

6. The method of claim 5, wherein the abnormal finding related to the abdominal disease comprise:
at least one of intraperitoneal hemorrhage, bowel ischemia, perforation, organ injury, free air, free fluid, vascular abnormality, inflammatory change, or mass.

7. The method of claim 2, wherein the method further comprises:
generating a suspected abnormal finding region map based on the predicted severity by using the first artificial intelligence model; and
generating an emergency reading report by using the medical image, the patient information, the emergency level, and the suspected abnormal finding region map.

8. The method of claim 7, wherein the first artificial intelligence model comprises:
an encoder configured to extract abnormal finding feature information from the medical image;
a classification module configured to calculate the severity based on the extracted abnormal finding feature information; and
a calculation module configured to generate a suspected abnormal finding region map based on the calculated severity.

9. The method of claim 7, wherein generating the emergency reading report by using the medical image, the patient information, and the suspected abnormal finding region map comprises:
displaying the suspected abnormal finding region map overlaid on the medical image in a form of a heatmap of color or contour; and
additionally displaying the severity and the emergency level.

10. The method of claim 7, wherein the method further comprises:
detecting a modification of the patient information while the emergency reading report is displayed;
re-triaging the emergency level based on the severity and the modified patient information; and
dynamically reflecting the re-triaged emergency level on the report in response to the modification of the patient information.

11. The method of claim 2, wherein the medical image comprises three-dimensional volume data composed of a plurality of slices, and
wherein the first artificial intelligence model is configured to detect the abnormal finding by learning a spatial context of the three-dimensional volume data.

12. The method of claim 1, wherein triaging the emergency level based on the predicted severity and the patient information comprises:
triaging into one of a surgical emergency grade or a conservative treatment possible grade.

13. A computer program stored in a non-transitory computer-readable storage medium, wherein, the computer program causes one or more processors to perform the following operations for triaging an emergency level of an abdominal disease by using an artificial intelligence model when the computer program is executed by the one or more processors, the operations comprising:
an operation of obtaining a medical image and patient information;
an operation of predicting a severity of the abdominal disease by using the medical image; and
an operation of triaging an emergency level based on the predicted severity and the patient information.

14. A computing device comprising:
at least one processor; and
a memory,
wherein the at least one processor is configured to:
obtain a medical image and patient information;
predict a severity of the abdominal disease by using the medical image; and
triage an emergency level based on the predicted severity and the patient information.
